Europäisches Patentamt

European Patent Office

Office européen des brevets

(11) Publication number: **0 322 922**
**A2**

## EUROPEAN PATENT APPLICATION

(21) Application number: **88121893.7**

(22) Date of filing: **30.12.88**

(51) Int. Cl.⁴: **G01N 33/569 , G01N 33/543 , G01N 33/577 , C12Q 1/00 , C12Q 1/68**

(30) Priority: **31.12.87 EP 87119416**

(43) Date of publication of application:
**05.07.89 Bulletin 89/27**

(84) Designated Contracting States:
**DE FR GB IT NL**

(71) Applicant: **Max-Planck-Gesellschaft zur Förderung der Wissenschaften e.V.
Bunsenstrasse 10
D-3400 Göttingen(DE)**

(72) Inventor: **Mölling, Karin, Prof.Dr.
Ihnestrasse 43
D-1 Berlin 33 (Dahlem)(DE)**

(74) Representative: **Vossius & Partner
Siebertstrasse 4 P.O. Box 86 07 67
D-8000 München 86(DE)**

(54) **Detection of avian and mammalian retrovirus infections.**

(57) The invention relates to a method of detecting a retroviral pol gene product in a sample, which comprises: (a) treating the sample with a disrupting agent to liberate the pol gene product, (b) contacting the treated sample of step (a) with a pol gene product-specific monoclonal antibody, to form a pol gene product/pol gene product-specific monoclonal antibody complex, (c) separating the complex of step (b) from the sample, and (d) determining the presence of the pol gene product either in the complex described in step (b) or after separation described in step (c).

Fig. 1

## DETECTION OF AVIAN AND MAMMALIAN RETROVIRUS INFECTIONS

Human T-cell lymphotrophic virus, type III (HTLV III), also designated lymphadenopathy-associated virus (LAV) or human immune deficiency virus (HIV), is the etiological agent of acquired immune deficiency syndrome (AIDS). HIV belongs to the group of viruses known as retroviruses. Retroviruses are characterized by a specific replication mechanism mediated by a virus-specific RNA-dependent DNA polymerase, also known as reverse transcriptase. This enzyme transcribes the viral RNA into a double-stranded DNA copy of the genome, called DNA provirus, Reverse transcriptase (RT) does not usually occur in normal cellular processes and does not normally exist as a cellular enzyme. Reverse transcriptase is not present in other RNA-containing organisms and is therefore considered unique to avian and mammalian retroviruses, such as, for example, HTLV-I, II, BLV, HIV and Hepatitis B.

In the simplest case, avian and mammalian retroviruses contain a limited set of genes: namely, the gag gene, encoding structural proteins; the pol gene, encoding the reverse transcriptase which catalyzes the DNA complement; and the env gene, encoding the envelope glycoproteins.

The avian and mammalian retroviral pol gene encodes not only the viral RNA-dependent and DNA-dependent DNA polymerase, but also the enzyme RNase H. These enzymes are defined here as pol gene products. The pol gene, especially the sequence encoding the carboxy-terminal RNase H domain, is a particularly highly conserved region among the various retrovirus isolates.

In the case of HIV the RT is a 66 Kd polypeptide (p66) which is often accompanied by a proteolytic cleavage product of 51 Kd (p51). The p51 product shares the common amino terminus but is truncated at the carboxy-terminus and, in contrast to the p66 molecule, the p51 molecule is enzymatically inactive. Both of these proteins are present in equal molar amounts in retrovirus particles.

The transcription of retrovirus-specific RNA into a double-stranded DNA provirus requires the help not only of the RT which transcribes RNA into an RNA-DNA hybrid, but also of an RNase H which specifically degrades RNA in the RNA-DNA hybrid. Retrovirus specific RNase H was first discovered by Moelling, et al. (Nature, New Biology, 234:240, 1971) in an avian retrovirus and was later identified as part of a single polyprotein comprising both RT and RNase H (Moelling, Virology, 62: 46-59, 1974; Journal of Virology, 18: 418, 1976).

The biological role of the RNase H is to catalyze the degradation of viral RNA at the 5' end of the genome, which after the onset of DNA synthesis by RT consists of an RNA-DNA hybrid. The viral RNA of this RNA-DNA hybrid is hydrolyzed by the virus-specific RNase H (Friedrich and Moelling, Journal of Virology, 31:630, 1979). The mode of action of the RNase H required for this step is that of a processive exonuclease, which means that the enzyme only catalyzes the degradation at the end of an RNA-DNA hybrid, not in the middle of the molecule. This degradative specificity is different from all other known RNase H activities in mammalian cells and in E. coli which have been characterized as endonucleases.

The p66 polyprotein comprising RT and RNase H activities has also been isolated from HIV particles. This Rnase H is also a processive exonuclease and therefore corresponds in its enzymatic properties to the previously described RNase H of other mammalian retroviruses. A 15 Kd protein (p15) which also exhibits RNase H activity has been identified as a cleavage product of the p66 polyprotein. The remaining truncated polyprotein is p51, which is enzymatically inactive both as reverse transcriptase and RNase H. The p15 protein is derived from the carboxy-terminus of p66.

Existing diagnostic tests for retroviral infections such as AIDS function primarily by detecting antibodies to the retrovirus. A major drawback to these types of tests, especially AIDS, is that antibodies are not produced in infected individuals until about 1 to 2 months after infection at the earliest, sometimes only about 6 to 16 months after infection and in some individuals apparently not until much later in the disease. In the later stages of AIDS antibody titers decline again while the virus is still present. Furthermore, the individual antibody response in an infected person or animal varies and gives rise to a high rate of so-called false-positive results. Therefore, second independent confirmatory assays, for example, Western blot analysis, are required before a person can be considered virus-positive and advised as such. Thus, considerable need exists for an AIDS test which can detect early stages of infections which does not rely upon the presence of anti-retroviral antibodies in the patient.

The detection of the retrovirus-specific RT and/or RNase H according to the present invention in individuals infected with HIV or other retroviruses is a highly sensitive antigen test compared to the widely used antibody tests for HIV which are based on the detection of polyclonal antibodies (antisera) in infected individuals. The RT and/or RNase H assay of this invention usually cannot be directly performed using body fluid, for instance blood from infected individuals, because inhibitory proteins and later, after infection, antibodies may be present in the body fluid which can inhibit the enzyme activities. Furthermore, the RT and/or RNase H may be present in a soluble state in the body fluid that is to be tested, since it is often present in the interior of virus particles or infected cells, or complexed to antibody. Therefore, it is an essential step in the assay method of this invention to disrupt preexisting structures and/or complexes, for example, by treatment with detergent-containing salt solutions or buffers with high or low pH, to dilute the solution obtained to adjust the pH and to isolate the p66 polyprotein comprising RT and RNase H using an immunoabsorption method to absorb these enzymes. Excess antibodies on the immunoaffinity material competes with the patient's antibody, if any is present, and displaces it because of the high molar excess. Therefore the RT and/or RNase H assay described here is not only applicable to HIV infected individuals before any antibody response occurs, but also at any later stage in the development of the disease.

A major object of the invention is to provide a novel and reliable method for detecting RT and/or RNase H molecules or activity of avian and mammalian retroviruses, for example, HTLV-I, HTLV-II, BLV, HIV or Hepatitis B, in body fluids such as blood, serum, urine, saliva, tears, semen and liquor from infected individuals or mammals.

The invention utilizes monoclonal antibodies to a retrovirus-specific RT and/or RNase H comprising pol gene product of avian or mammalian origin. These monoclonal antibodies can be directed against any epitope of the p66 polyprotein comprising RT and RNase H as long as they do not inhibit one or both of these activities.

A further object of the invention is to detect RT and/or Rnase H molecules or activities immobilized in immune complexes.

Another object of the invention is to immobilize the p66 polyprotein using monoclonal antibodies which inhibit the RT and/or RNase H enzymatic activities and to elute the p66 polyprotein without destroying the enzymatic activity of RT and/or RNase H.

Yet another object of the invention is to provide a kit for the detection of retrovirus infections in birds or mammals based on the following reaction:

$$RT + MAB^i + 3dXTP + ldXTP^* + matrix \xrightarrow[\substack{\text{temperature,} \\ \text{1 to 20 hrs}}]{\text{RT buffer}} DNA^*$$

dXTP = one of the four deoxynucleoside triphosphates
MAB$^i$ = immobilized monoclonal antibody
dXTP$^*$ = labeled deoxynucleoside triphosphates
DNA$^*$ = labeled DNA

The immune complex-bound retrovirus-specific RT and/or RNase H assays described here have the following advantages:

(1) The assay can detect a retrovirus infection prior to antibody response, that is, very early in viral replication. The assay can therefore be used to make a diagnosis much earlier than is possible using the assays known from the prior art.

(2) The assay can be performed with donor fluid, such as blood, and can detect viruses at an early stage of infection, that is, prior to antibody production. Therefore, the assay can be used to reduce the risks involved in the transfusion of contaminated blood.

(3) Since the assay is an antigen test it is based on a different principle than all other tests presently available, which are antibody tests. Thus, the assay of the invention can be used as an independent or confirmatory test.

(4) The assay can detect any avian or mammalian retrovirus that may arise by genetic variation, since the pol gene is a particularly highly conserved region of the entire retroviral genome, in contrast to the env gene, for instance.

(5) The assay can be set up in such a way that it detects unknown viruses that might arise or already exist. For this purpose monoclonal antibodies to the most highly conserved epitopes of the pol polyprotein are preferred. If desired, several monoclonal antibodies of differing specificity can be used in combination as a "cocktail" to detect RT and/or RNase H.

(6) The assay is more sensitive than any antigen or antibody assay known today. It recognizes at least 0.1 picogram of RT molecules in 0.1 ml serum in a standard assay using $^3$H-dTTP (see Examples 3, 4 and 5, infra). The assay can be made even more sensitive, for example, by using $^{32}$P-labeled deox-

3

ynucleoside triphosphates, longer incubation periods (the reaction is linear for more than 20 hours), or greater quantities of starting material. The assay can be modified using labels other than radioactivity commonly known to those of ordinary skill in the art.

(7) The assay does not require infectious material to be handled. When a blood sample is taken from a patient the sample can be concurrently treated with a nonionic detergent which will completely destroy the infectivity of the virus without eliminating enzyme activity. Further treatment by heat (56°C, 30 min) as a standard inactivation treatment of infectious material reduces enzymatic activity by no more than about 5%. Also, the samples can be sent by regular mail to reference laboratories or stored frozen.

(8) The assay is simple and quick and can be used to monitor the effect of chemotherapy in patients during treatment, since it is a direct measure of the number of virus particles. Also, the assay is quantitative and can be easily standardized.

(9) The assay can be used to determine the effectiveness of inactivation of so-called live viral vaccines.

(10) The assay is superior to the newly developed polymerase chain reaction (PCR) in that only infectious virus is monitored and because it is quantitative and standarized. The PCR method detects also non-infectious defective particles or defective DNA proviruses which do not lead to disease. The PCR method is also difficult to standardize or quantitate and is also hampered by the genetic variability of the retroviral genome to a larger extent than the assay described here. The PCR may be too sensitive a test in that it detects even rudimentary DNA regions.

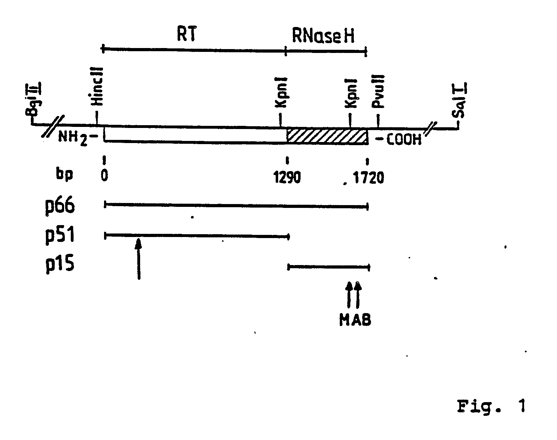

Figure 1 is a schematic drawing of part of the HIV-polymerase gene (Ratner et al., Nature, 313: 277, 1985), showing the RT and the RNase H regions. The Kpnl (left and right) and PvuII restriction sites used for epitope mapping of the monoclonal antibodies (MAB) are indicated. The recognition site of three MABs obtainable according to the the foregoing procedure is indicated by arrows. The hatched area defines the location of the RNase H. Proteins p66, p51 and p15 are indicated by straight lines.

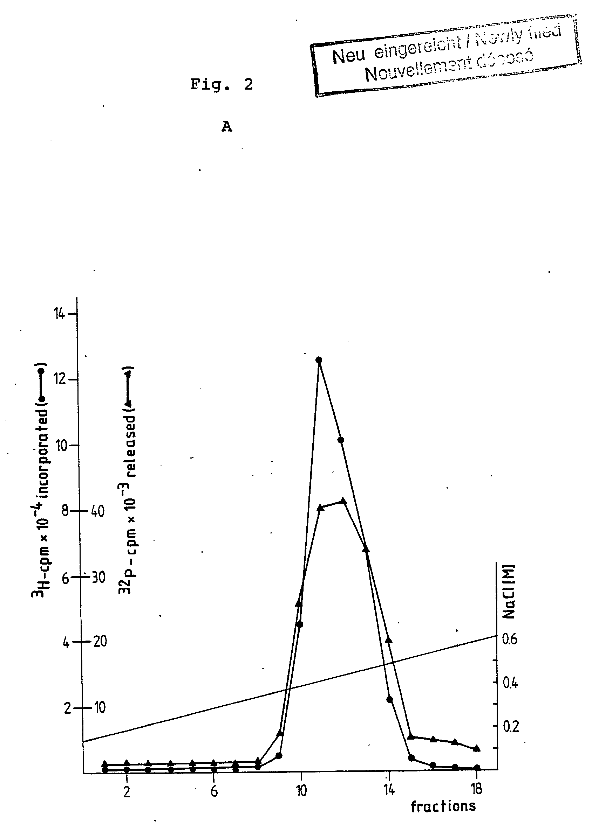

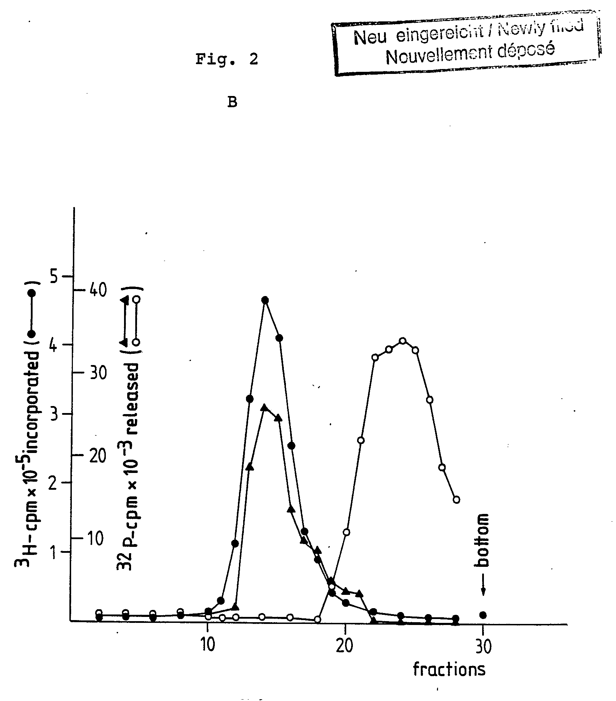

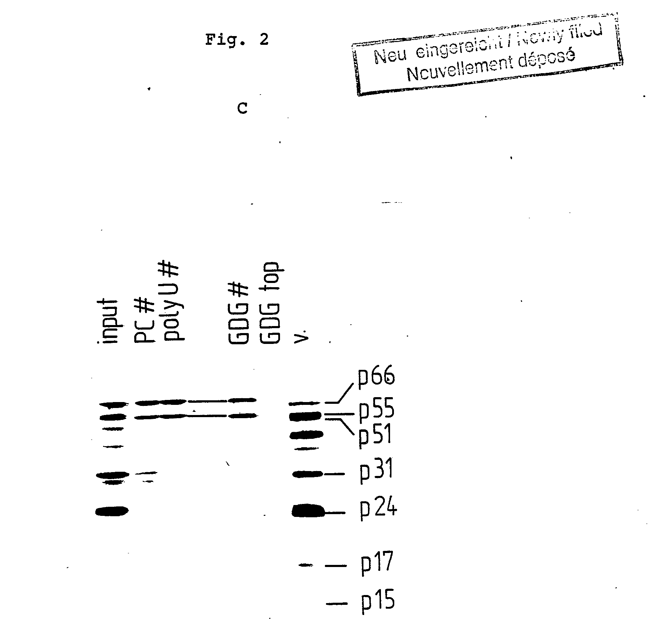

Figure 2A illustrates the purification scheme of the p66 polyprotein comprising RT and RNase H from HIV virus particles. Lysed virus was applied to a DEAE-cellulose column. The flow-through was applied to a phosphocellulose column. The p66, eluted with 0.2 M NaCl, was further processed on poly(U)-Sepharose® column. The elution profiles of p66 associated RT (1) and RNase H (2) activities from HIV virus from poly-(U)-agarose column are shown. Elution profiles from the corresponding recombinant enzymes, which were prepared by genetic manipulation, are shown in Hansen et al. (Journal of Biological Chemistry, 262: 12393, 1987, Figures 2A and B).

Figure 2B shows reverse transcriptase and RNase H assays of glycerol density gradient fractions. Two samples were centrifuged in parallel: fraction 11 shown in Figure 1A, and the flow-through material at 0.1M NaCl from the poly(U)-Sepharose® column. Reverse transcriptase (1), RNase H (2) of fraction 11 and RNase H (3) of the flow-through are indicated. "Bottom" indicates the material that was resuspended sediment from the tube.

Figure 2C shows immunoblots of HIV-lysate used as starting material (labeled "input"), eluted fraction 4 of the PC column, fraction 11 of poly(U)-Sepharose®, and fractions 14 and 24 (labeled "top") from the glycerol density gradient (GDG). Patient serum was used in 1:500 dilution. v : viral protein marker. A 12.5% polyacrylamide gel was used.

Reverse transcriptase (RT) can be present in body fluids either as a component of virus particles, intracellularly, or as part of a pre-existing antigen-antibody complex. RT can be liberated from such components or complexes by using a viral disruption agent. A typical example of a disruption agent is a 0.2 to 2% solution of Nonidet® Np-40 or Triton® X-100, or other non-ionic detergents containing 1 to 4 M $MgCl_2$, or a 1 to 2M KSCN solution or other salt solution in an otherwise physiological buffer (typically, 50 M Tris-HCl, pH 7.8) or buffers with low pH (for example, citrous acid, pH 3.5) or high pH (for example, pH 10.5). The salt is diluted out or the pH brought close to neutral (for example, pH 8.5) before contacting it with the immobilized MAB. In order to conserve enzymatic activities 1 mM dithiothreitol (DTT) or other reducing agent is usually added for, typically, 10 to 20 min at 4°C. Subsequently, debris is removed, for example, by centrifugation in an Eppendorf centrifuge (10,000 rpm, 5 min, 4°C) and the clarified supernatant containing the soluble enzyme is contacted with a monoclonal antibody (MAB) directed to a retroviral specific pol gene product (polyprotein) comprising reverse transcriptase (RT) and/or RNase H.

The MAB used in the assay of the invention may be directed against any epitope of the pol gene product comprising RT and/or RNase H of avian or mammalian retroviruses. The MABs may be produced by conventional procedures well-known in the art (Galfre and Milstein, Methods in Enzymology, 73: 3, et seq., 1981).

4

The antigen used for immunization can be obtained either from retroviral components or recombinant retroviral proteins of avian or mammalian origin expressed, for example, in E. coli or other microorganisms or in eukaryotic cells. In the present invention, a recombinant retroviral pol gene product was produced by expression of HIV-DNA (HincII/KpnI, right fragment) in E. coli (Fig 1). Three MABs directed against this protein were prepared in a conventional manner (indicated by arrows). One monoclonal antibody (cl 8/6) is directed against an amino-terminal epitope of the pol gene product, the other two monoclonal antibodies, designated cl 6/23 and cl 9/5, are directed against a carboxy-terminal epitope of the pol gene product. The carboxy-terminus of the pol gene product comprises the RNase H. Both of these latter monoclonal antibodies recognize the RNase H in the p66 HIV-specific polyprotein as well as the pl5-RNase H. These two RNase H activities are novel in HIV. Expression of HIV DNA in E. coli yields a p66 polyprotein exhibiting RT and RNase H activities (Hansen et al., Journal of Biological Chemistry, 262: 12393, 1987).

Selection of MAB-producing hybridomas is performed, for example, using the antigen, for example, produced as described above, in an ELISA screening test or using Western blots or indirect immunoprecipitation of the antigen from radioactively labeled bacterial or HIV-infected mammalian cell lysates. Positive hybridoma clones are subcloned using standard techniques until each stably produces only one immunoglobulin. The MABs from positive hybridomas are then characterized to determine whether they inhibit the RT and/or RNase H activity. Such tests can be performed by immunoprecipitation of active RT and/or RNase H with the MAB and subsequent ELISA or standard RT and/or RNase H assays. MABs which inhibit RT and/or RNase H activity will block detection of the reaction products of these enzymes. Also, at this stage the isotypes of the MABs may be determined. MABs of the $IgG_{2A,B}$ or IgG, isotypes are preferred as compared to IgM isotype because they typically bind more efficiently to Protein A or Protein G carriers. The determination of the isotype follows standard techniques using commercially available kits (for example, from Boehringer Mannheim or Sigma Co.).

Mass production of MABs can be performed by ascites production following standard techniques (Galfre and Milstein, supra). Typically, mice are pretreated twice with 0.1 ml Pristane® intraperitoneally (ip) and then receive $10^8$ to $10^8$ hybridoma cells. After 2 to 3 weeks ascites fluid develops and is drawn off by a syringe. The ascites fluid can be stored frozen. Alternatively, MAB mass production can be performed in cell culture with harvesting of the culture supernatants.

Immobilization of MAB can be achieved in accordance with conventional methods by binding MAB to a carrier. A preferred binding carrier is CNBr-activated Sepharose®. An even more preferred carrier for immobilizing the MAB is Protein A or Protein G-Sepharose® (Pharmacia Co.). Contacting a supernatant or ascites fluid containing MAB with the Protein A or Protein G-Sepharose® beads for about 30 min at about 4°C will bind the MAB. To stabilize the MAB on the column, for example, if it is washed to regenerate the column material and reuse it, the MAB can be coupled in accordance with standard techniques (Donner et al., Nature, 296:262, 1982) or as indicated by the manufacturer (Pharmacia Co.). The MABs coupled to carrier are termed immobilzed MAB. The immobilized MAB can be put, for example, into mini columns, such as blue Eppendorf tips. This material is referred to as an immunoaffinity column and allows rapid passage of large volumes for wash procedures and therefore results in very efficient purification (up to 3000 fold in a single elution step).

the supernatant containing the soluble polyprotein comprising RT and/or RNase H is usually diluted (about 5 to 10 fold) using an RT buffer, for exmaple, 50 mM Tris-HCl, ph 8; 1 mM $MgCl_2$, 80 mM KCl, 1 mM DTT, 0.02 Nonidet® NP-40 which can also be supplemented with 10% glycerol and then placed in contact with the immobilized MAB. The contact can be performed in a sealed plastic tube containing the diluted supernatant and the immobilized MAB. Shaking for about 10 to about 30 min at about 4°C will ensure binding of the pol gene product to the immobilzed MAB. This material can then be packed in a minicolumn, for example, a blue Eppendorf tip.

To remove non-specific components the mini-column is washed with seveal ml of RT buffer. The resulting material bound to immobilized MAB consists of polyprotein comprising RT and/or RNase H activity. This material, or an aliquot thereof, is resuspended in a small volume of RT buffer (for example, 0.1 to 0.2 ml) and used directly in an RT assay as long as teh MAB was one that does not inhibit the enzymatic activity of the pol gene product.

The reverse transcriptase (RT) assay for detecting the presence of the RT enzyme requires the four deoxyribonucleotides dATP, dGTP, dCTP and dTTP, also denoted as dXTP, for the preparation of DNA complementary to the matrix. Detection of the enzymatic reaction products requires detectably labeling at least one, preferably two, and most preferably all four, deoxyribonucleotides. The dXTP can preferably be detectably labeled by radiolabeling with $^3$H or, for a more sensitive test, with $^{32}$P, for example, $^3$H-dTTP or $^{32}$P-dTTP. Alternatively, biotin/streptavidin or fluorescent agents or other tagging molecules can be used for labeling. The sensitivity of the reaction can be improved if the labeled deoxyribonucleotide is not diluted with the same unlabeled deoxyribonucleotide. The optimum concentration is reached when the labeled deoxyribonucleotide is not limiting the polymerization reaction.

Reverse transcription requires a matrix or template which is transcribed. In the infected cell the template consists of the natural viral RNA which carries a starter molecule, for example, a tRNA primer. This is a requirement for the enzyme to initiate the polymerization reaction. In vitro any natural RNA, not necessarily of viral origin, for example, globin mRNA, can be used as the template. The primer can also be a short oligonucleotide fragment that binds to the mRNA. Typically oligo(dT)$_{10}$ is used, which binds to the poly(A) tail present at the 3' end of all mRNAs. Alternatively, synthetic template primers can be used. A typical example is poly(rA)$_{100-200}$ • oligo(dT)$_{10}$ (Boehringer Co., Tutzing). While the length of the poly(rA) strand is not critical, it should allow synthesis of DNA fragments long enough for separation from the deoxyribonucleotides, for example, from about 10 to 20 and up to about 200. The length of the primer is not critical, but it should preferably be shorter than the template, for example, by a factor of 5 to 10. An example of a typical primer is oligo (dT)$_{10}$. Alternatively, other synthetic template primers can be used, such as poly(rC) • oligo(dG). Some reverse transcriptases very specifically copy methylated forms, such as poly(rC)m • oligo(dG), which are not accepted by cellular DNA polymerases and potentially contaminate reverse transcriptase preparations. Also, the ability of reverse transcriptase to synthesize DNA from "nicked", that is, exhibiting single-stranded breaks, double-stranded DNA can be exploited for a sensitive assay. A typical example is "nicked" calf thymus DNA (Boehringer-Mannheim). The reverse transcriptase reaction starts at the ends of the nicks and therefore does not require any additional primers.

The reaction buffer for reverse transcription is the same buffer mentioned above as RT buffer. The reaction volume is typically about 100 microliters. Alternative buffer recipes can be found in Maniatis, et al. (Molecular Cloning - A Laboratory Manual, Cold Spring Harbor Press, 1982). The divalent cation requirement, usually $Mg^{2+}$ or $Mn^{2+}$, depends on the species from which the virus originates and is easily optimized according to the specific case under investigation. For example, avian retrovirus RT prefers 10 mM $Mg^{2+}$, murine retrovirus Rt 0.1 mM $Mn^{2+}$ and HIV RT prefers 5 mM $Mg^{2+}$.

Incubation temperature is normally 37°C, except for avian RT, which works optimally at 41°C which is the average body temperature of birds. Incubation periods depend on the sensitivity required. The RT is stable enough for synthesis for at least 10 to 20 hrs at 37°C. A typical incubation period is 1 to 2 hrs.

Determination of the reaction product requries separation of the deoxyribonucleotides incorporated into DNA from those that have not been incorporated. In the case of radiolabeled deoxyribonucleotides, the labeled synthesized DNA is precipitated with acid (for example, 10% trichloroacetic acid, 30 min at 4°C) and then filtered off. The labeled and incorporated DNA remains on the filter, whereas the non-incorporated deoxyribonucleotides flow through the filter. The amount of labeled DNA is determined by a detection system such as, for example, a scintillation counter or an autoradiographic film and is proportional to the amount of RT enzyme which, in turn, is proportional to the number of retroviruses. If a filter assay is used, it is preferred to allow the immobilized MAB enzyme complex to sediment and to analyze an aliquot of the soluble supernatant.

In the case of a MAB which inhibits and/or RNase H activity the procedure is as follows:

$$\text{RT + MAB} \xrightarrow{\text{elution solution}} \text{RT}$$

$$\text{RT + 3dXTP + 1dXTP* + matrix} \xrightarrow[\substack{\text{temperature,}\\\text{1 to 20 hrs}}]{\text{RT buffer}} \text{DNA*}$$

An additional step is necessary. The immobilzed MAB is contacted with the polyprotein comprising RT and/or RNase H. The inhibition of enzyme activity by MAB means that the polyprotein needs to be separated by an elution agent. Any conventional elution agent may be used that does not destroy enzyme activities. Typical elution agents are chaotropic agents, or buffers of low pH or high pH. Specific buffers include 4 M $MgCl_2$ or 0.5 M to 2 M KSCN or citric acid buffer at pH 3.5. The buffer is applied to the complex of the immobilized MAB and RT, incubated for 10 to 30 min at 4°C and subsequently neutralized if necessary. The clarified supernatant, for example, obtained after low speed centrifugation, contains the RT. An aliquot of this solution representing approximately 10% of the RT reaction volume is complemented with RT buffer, 3dXTP, labeled dXTP and matrix. All further steps are carried out as described above for non-inhibiting MAB. Alternatively, the eluted RT can be dialyzed in RT buffer to remove the elution buffer. The detection of RNase H is as follows:

$$\text{RNase H + RNA-DNA hybrid* } \xrightarrow[\substack{\text{temperature} \\ \text{10 to 60 min}}]{\text{RT buffer}} \text{ nucleotides* and/or residual RNA-DNA hybrid*}$$

\* radiolabeled or otherwise labeled.

All procedures are performed as described above for RT with the following exceptions: the RNase H assay differs from the RT assay since it is a hydrolyzing (degradative) enzyme, in contrast to the RT which is a polymerizing enzyme. Therefore, the RNase H requires a preformed substrate, here, the RNA-DNA hybrid, that can be hydrolyzed. The RNA-DNA hybrid needs to be labeled, in particular the RNA strand. The labeling can be performed by radioactivity, biotin/streptavidin, fluorescence or using other substances. Hydrolysis of the labeled RNA from the RNA-DNA hybrid starts at the free end of the hybrid, since retroviral RNase H activities are typically exonucleases - in contrast to cellular RNase H which cleaves the RNA-DNA hybrid endonucleolytically. Typical reaction products of hydrolysis of RNA-DNA hybrids are mono-, di- and small oligoribonucleotides which can then be detected. For example, the released nucleotides can be separated from the residual undegraded RNA-DNA hybrid, which is precipitated by acid treatment, for example, using 10% trichloroacetic acid. The precipitate is collected on a filter and the hydrolyzed nucleotides are washed away or collected in the filtrate. Alternatively, after acid treatment the hybrid can be centrifuged to the bottom of the tube and an aliquot of the supernatant, containing hydrolyzed labeled nucleotides, can be determined by liquid scintillation counting. The amount of hydrolyzed nucleotides reflects the amount of RNase H activity. The amount of residual RNA-DNA hybrid is inversely proportional to the amount of RNase H. A typical RNase H reaction lasts 15 to 60 min, because normally the amount of RNA-DNA hybrid is exhausted by then.

The preparation of RNA-DNA hybrids follows standard procedures using any DNA and cellular RNA polymerase and radiolabeled or otherwise labeled ribonucleotides for synthesis of RNA*-DNA hybrids. Typical reactions are described in Maniatis, et al. (supra). Alternatively, synthetic RNA-DNA hybrids may be purchased or synthesized, for example, poly(dT)•poly(rA), wherein the length of the two polymers is not critical. Another typical exmaple is poly(dT)$_{100}$•poly(rA)$_{100}$, in which the poly(rA) is labeled with $^3$H or $^{32}$P. This labeled molecule is commercially available.

The above disclosure generally described the present invention. A more complete understanding can be obtained by reference to the following specific examples which are provided herein for purposes of illustration only and are not intended to limit the scope of the invention.

EXAMPLE 1

Preparation and isolation of monoclonal antibodies directed against the pol gene product

The pol gene of HIV ws cloned into an expression vector using a BglII/SaII DNA fragment (see Fig. 1) and inserted into a derivative of pPLc24 (Bading, et al., Oncogene, 1:395, 1987). This vector is thermoinducible and contains a polylinker with restriction sites for cloning. Plasmid ptrp9 may also be used as the expression vector (Hansen, et al., supra).

The resulting protein expressed in E. coli is a fusion protein, MS2-pol, containing the first 99 amino acids encoded by the replicase gene of bacteriophage MS2. The MS2-pol polyprotein is about 160 Kd in size and is degraded by bacterial proteases in 20 min to the RT molecule, which is 66 Kd in size, and to the truncated p51 protein (Hansen, et al., supra). The p66 antigen is eluted from SDS polyacrylamide gels concentrated by gel elution using a Biotrap® (Schleicher & Schuell) or purified as described (Hansen, et al., supra) and used to immunize mice. The immunized mice, in turn, serve as a source of B-cells for the production of hybridomas secreting monoclonal antibodies. These MABs can be used, for example, in the ELISA or Western blot techniques.

The production of monoclonal antibodies follows published procedures (Galfre and Milstein, supra, 1981; Bading, et al., supra). Hybridoma cells producing immunoglobulins were repeatedly subcloned to guarantee that they produce only one monoclonal antibody. Bacterially expressed subfragments of the BglII/SalI fragment allow epitope mapping of the monoclonal antibodies produced by the hybridoma (Fig. 1). Two hybridoma cell lines in these routine experiments, cl 6/23 and cl 9/5, were identified as producing MABs that recognize the p66 protein, but not the p51 protein in HIV proteins. The monoclonal antibodies of one clone (cl 6/23) recognize a KpnI(left)/PvuII expression protein as well as a KpnI(right)Sa1II expression protein. These MABs thus recognize the region between KpnI (right) and PvuII (the restriction map is taken from Ratner, et al., supra). Another monoclonal antibody (clone cl 8/6) recognizes the p51 and p66 proteins, and the corresponding epitope is located at the amino-terminus of pol gene product. The production of ascites follows published procedures (Galfre and Milstein, supra). Mice pretreated with Pristane® are injected with $10^8$ to $10^7$ hybridoma cells, which produces ascites within one to two weeks. Ascites fluid is recovered from the mice using syringes and stored frozen. Since the antigen, that is, the p66 protein, can be prepared by recombinant DNA technology (Hansen, et al., supra), deposition of hybridomas as a substitute for an enabling dislosure for the preparation of MABs directed to that protein is not necessary.

## EXAMPLE 2

Alternative cloning procedures were applied to the DNA restriction fragment HincII/PvuII and to HincII/PvuII in which the KpnI (left) and KpnI (right) restricted fragment had been omitted. Thus, in the second case an internal portion of the pol gene is deleted. This results in a truncated gene product of 50 kd (p50). The resulting proteins expressed in E. coli were in in the first case identical to the ones described in Example 1, namely p66 and p51. While the p66 polyprotein exhibited RT and RNase H activities, the p50 no longer exhibited RNase H activity and showed an alteration of the RT in that it did not bind efficiently to the poly(U) Sepharose column (Fig. 2A) (data not given here).

## EXAMPLE 3

### Characterization of RT and RNase H from HIV virus particles

HIV was propagated and purified under standard conditions in H9 cells (Popovic, et al., Science, 224:497, 1984). The virus ws inactivated by treatment with 2% Nonidet® NP-40 detergent for 10 min at 4°C and stored frozen. This treatment completely destroys the viral architecture and its infectivity. Before use the lysate was further inactivated by heat treatment (56°C for 30 min). This treatment totally destroys the viral RNA and viral infectivity. Both RT and RNase H activity, however, can still be detected in the lysates. Both activities are contaminated by cellular DNA polymerases and cellular RNase H activities. Purification of virus-specific activities is therefore required. The enzymes copurify during conventional column chromatography using DEAE cellulose, phosphocellulose,poly(U)-Sepharose, adn glycerol density gradient centrifugation (Hansen, et al., supra).

The purified RT activity was characterized using poly(rA)•oligo(dT) template primer, which mimics the RNA templates and allows DNA synthesis. An RNA-DNA hybrid with a radiolabeled RNA served as the substrate for detection of RNase H. These results are illustrated in Fig. 2A, B, C.

## EXAMPLE 4

### Reverse Transcriptase and RNase H assays

Each fraction was analyzed for enzyme activities. Reverse transciptase was tested in a 100 μl assay consisting of RT buffer (50 mM Tris-HCl, pH8, 1 mM MgCl₂, 80 mM KCl, 1 mM DTT, 0.02% Nonidet® NP-40), 1 μg of poly(rA)•oligo(dT)₁₀, [³H] TTP (5 μl, 50 Ci/mmol, 1 mCi/ml), and 5 to 10 μl of enzyme solution.

Incubation was carried out for 60 min at 37°C. The reaction was stopped by the addition of 2 ml of 10% trichloroacetic acid, followed by incubation for 20 min at 4°C. The acid-precipitated material was collected on a filter (GF/C, Whatman) and washed 5x with 5 ml of 10% trichloroacetic acid. The non-specific filter background never exceeded 1000 cpm. RNase H was analyzed by assaying 100 µl using the RT buffer described above and 25,000 cpm of the hybrid. The hybrid consisted of $^3$H-poly(rA) ($10^4$ cpm/pmol, 4 z $10^3$ cpm/µl) and poly(dT). Alternatively, to obtain a more sensitive hybrid, the hybrid was transcribed from single-stranded mp8 DNA (Bethesda Research Laboratories) using E. coli RNA polymerase with [$^{32}$P]UTP, to produce a hybrid of $10^8$ cpm/pmol and 2 x $10^3$ cpm/µl. RNase H assays were incubated for 15 min at 37°C. The acid-precipitated radioactive hybrid remaining was determined as above. The radioactivity released from the hybrid was determined by subtracting the value of the residual hybrid from the value of the undigested RNA-DNA hybrid starting material.

## EXAMPLE 5

### Determination of RT and/or RNase H in an immune complex

Enzymatically active RT and/or RNase H was purified as published (Hansen, et al., supra) and used to test immobilzation in immune complexes and to determine the residual activities. The same methodology was used for immobilizing the RT and/or RNase H. 1 ml of ascites fluid obtained according to Example 1 was bound to 1 ml of Protein A-Sepharose® beads (Pharmacia Co., Sweden). Covalent coupling with Suberimidate® may be performed, but is not required (Donner, et al., Nature, 296:262, 1982). The immunoglobulin coupled to the Protein A-Sepharose® beads was washed with phosphate-buffered saline (PBS) containing 0.1% Nonidet® N P-40 and resuspended in 1 ml of the same buffer. Enzyme or virus lysate was added to these washed immunobeads and allowed to bind at 4°C for 10 min. The immunobeads were washed again and sedimented by low speed centrifugation to remove non-specific components (3 x 1 ml PBS plus 9.2% Nonidet® NP-40). The sedimented immune complex contained the RT and/or RNase H, which was directly suspended in RT assay buffer. Radioactive deoxyribonucleotides or radiolabeled RNA-DNA hybrid was added to allow polymerase or RNase H reactions to occur. The results of the determination of p66-RNase H and p15-RNase H activities by this method are summarized in Table 1.

Table 1

| Immunoprecipiation of RNase H by monoclonal antibody | | | |
|---|---|---|---|
| | | $^{32}$P-cpm released (%) | |
| ANTIBODY | Vol. | GDG # 15 | GDG #24 |
| hybridoma | 10µl | 15,000 (24) | 19,000 (28) |
| hybridoma | 30µl | 29,000 (43) | 34,000 (51) |
| NS-1 | 10µl | <300 (0) | <300 (0) |
| NS-1 | 30µl | <300 (0) | <300 (0) |
| RS | 10µl | 6,000 (10) | 10,000 (25) |
| RS | 30µl | 13,000 (21) | 19,000 (28) |
| GDG = glycerol density gradient | | | |
| NS-1 = myeloma supernatant | | | |
| RS = rabbit anti p66 serum | | | |
| Figures in parentheses denote percentage of hybrid degraded. | | | |

Supernatant of a hybridoma producing antibody to the terminal epitope of the bacterially expressed p66 RT molecule was coupled to Protein A-Sepharose® beads and used to immuno-precipitate the p66-RNase H from the fraction with the hybrid activity (10 and 30 µl from fraction 15) and the p15-RNase H from the top fraction (10 and 30 $\mu$l from fraction 24) of the glycerol density gradient (GDG) (see Figure 2B). These enzymes immobilzed in immune complexes were pelleted, resuspended in RNase H assay buffer and tested in standard RNase H assays. The radioactive hybrid precipitated after the reaction was subtracted from the total amount of undigested hybrid used as input (66,500 cpm corresponding to 100%) and is indicated as cpm released and as a percentage of the hybrid degraded by RNase H. NS-1 represents myeloma supernatant used as a control instead of hybridoma.


## EXAMPLE 6


## Determination of HIV specific RT in patient's blood by adsorption on immunobeads


Human serum (0.1 ml) taken from a pool of five individuals who tested positive in a conventional antibody test for anti-HIV antibodies was complemented to 2% Nonidet® NP-40 and 1 M $MgCl_2$, inactivated at 56°C for 30 min and stored frozen at -20°C. Before use the serum was diluted 1:10 with RT buffer and centrifuged (10,000 g, 4°C, 3 min). This material was loaded onto a blue Eppendorf tip used as a column and containing 0.1 ml of Protein A-Sepharose® beads covalently coupled to 0.1 mg of purified monoclonal antibody to the p66 polyprotein. The column was then washed 3 times with 1 ml of RT buffer. Seveal elution buffers were tested. A buffer of 1 to 1.5 M KSCN proved useful for elution. RT activity (80%) present in the starting material was recovered using this buffer. Three fractions of 0.1 ml each were eluted and analyzed immediately. An aliquot (10 $\mu$1) incorporated 23,000 cpm in 60 min at 37°C in a 100 $\mu$1 standard RT assay using $^3$H-dTTP (53 Ci/mmol, 5 $\mu$Ci/assay) without addition of unlabeled dTTP. When normal serum was similarly processed, the incorporation was 3000 cpm. No significant amount of DNA-dependent DNA polymerase activity, a potential contaminant from cellular debris, could be detected as evidenced by the use of poly(dA)•poly(dT) as the substrate. The data are summarized in Table II. The eluted enzyme can be stored at -20°C in 50% glycerol, 2mM DTT, and can be frozen and thawed several times or dialyzed without significant loss of activity.

Table II

| | AB positive serum (infected subject) | control serum |
|---|---|---|
| poly(rA).oligo(dT)$_{10}$ | 23,000* | 3,000 |
| poly(dA).poly(dT) | 4,005 | - |
| background | ≤ 1,000 | ≤ 1,000 |

*in CPM

A standard reverse transcriptase assay was performed with immunoaffinity purified enzyme (10 $\mu$1 or 100 $\mu$1 of eluted fraction N•1) using poly(rA)•oligo(dT)$_{10}$ as the template-primer. Poly(dA)•poly(dT) was used as a control for cellular DNA-dependent DNA polymerase activity (for the assay conditions see Hansen, et al., supra).


## EXAMPLE 7


## Assay of RT and/or RNase H molecules using monoclonal antibodies


A prerequisite for the identification of monoclonal antibodies to retrovirus-specific pol gene product is the formation of an antigen-antibody complex (pol protein-MAB complex). The formation of this complex is used, for example, in the ELISA screening test or Western blot in selecting MAB producing hybridomas.

These initial hybridoma screening assays do not involve a determination of the enzymatic activity of the pol gene coded reverse transcriptase and/or RNase H, but merely test for the presence of the pol protein. Reverse transcriptase and/or RNase H enzyme assays will only be required if further characterization of the MAB is desired to determine whether it inhibits the enzymatic activity of the pol protein.

The ELISA screening test can, however, also be used to find the pol protein once corresponding MABs are available. The pol protein-MAB complex can then be used as a test to find the pol gene product in animals or humans infected with retroviruses, In such a test a MAB to the retrovirus-specific pol gene product is immobilized, for example, on an ELISA plate. Serum from an infected individual, containing the pol gene product, is added to the immobilized first MAB on the ELISA plate so that the immobilized MAB recognizes and captures the pol gene product, as an antigen. It may be necessary -as described in Example 6 - to pretreat the serum with a virus disrupting agent to liberate the pol gene product from intact virus particles, infected cells, or with low or high pH buffer to liberate the RT from a pre-existing immune complex. Once the pol protein-MAB complex has formed, a standard wash buffer solution is applied to the complex to remove non-specific components.

A detection system is used to detect the pol protein MAB complex. For this purpose, a second MAB or a cocktail of several MAB (both designated $MAB_2$), directed to the pol protein can be used. $MAB_2$ preferably has a different epitopic specifity from the first MAB (designated $MAB_1$). $MAB_2$ can be coupled to a substance (S) which permits detection of the complex. Such as substance is, for example, an enzyme, such as lactoperoxidase or alkaline phosphatase, well-known for use in standard Western blot techniques. After washing the $MAB_1$-pol protein-$MAB_2$ complex, a buffer solution containing substrate is added. Typically, the enzyme substrate, for example, para-nitrophenylphosphate (PNPP), is enzymatically modified and produces a color. The reaction can be summarized as follows:

$MAB_1$ + (pol protein) + ($MAB_2$ - S) + PNPP → color

This assay is sometimes denoted an "antigen-capture assay", since it captures a viral antigen (in this case pol protein) from the body fluid of an infected individual. The detection system can alternatively be, for example, a streptavidin-biotin complex or a radiolabel. This assay is generally less sensitive than the assay described in Example 6, which detects enzyme activities of the reverse transcriptase and/or RNase H and allows the use of significantly greater volumes of body fluid than is possible in the antigen-capture assay.

The invention now being fully described, it will be apparant to one of ordinary skill in the art that many changes and modifications can be made thereto without departing from the spirit or scope of the invention.


## Claims

1. A method for detecting a retroviral pol gene product in a sample, which comprise:
   (a) treating said sample with a disrupting agent to liberate said pol gene product,
   (b) contacting said treated sample of step (a) with a pol gene product-specific monoclonal antibody, to form a pol gene product/pol gene product-specific monoclonal antibody complex,
   (c) separating said complex of step(b) from said sample, and
   (d) determining the presence of said pol gene product.

2. The method of claim 1 wherein said step (c) further comprises dissociating said pol gene product from said complex.

3. The method of claim 1 or 2 wherein said pol gene product specific monoclonal antibody is immobilized.

4. The method of any one of claims 1 to 3 wherein said determining the presence of said pol gene product is by measuring the enzymatic activity of said product.

5. The method of claim 4 wherein said enzymatic activity is due to reverse transcriptase (RT).

6. The method of claim 5 wherein said enzymatic activity is detected by the synthesis of a detectably labeled polynucleotide.

7. The method of claim 4 wherein said enzymatic activity is due to processive exonuclease (RNase H).

8. The method of claim 7 wherein said enzymatic activity is detected by the degradation of a detectably labeled polynucleotide.

9. The method of any one of claims 1 to 3 wherein said determining the presence of said pol gene product is by contacting said pol gene product with detectably labeled antibody.

10. The method of claim 9 wherein said detectably labeled antibody is a monoclonal antibody with epitopic specificity different from that of the monoclonal antibody of step (b).

11. The method as in either of claims 6 or 8 wherein said detectable label is selected from the group consisting of a radiolabel, a fluorescent label, biotin/streptavidin and an enzyme.

12. A kit useful for the detection of a retroviral pol gene product comprising a carrier being compartmentalized to receive in close confinement therein one or more containers wherein
   (a) a first container contains a pol gene product specific monoclonal antibody, and
   (b) a second container contains a detectably labeled deoxynucleotide.

13. The kit of claim 12 wherein said detectably labeled deoxynucleotide is part of an RNA polymer.

14. The kit of claim 13 wherein said RNA polymer is hybridized to DNA.

15. A kit useful for the detection of a retroviral pol gene product comprising a carrier being compartmentalized to receive in close confinement therein one or more containers wherein
   (a) a first container contains a pol gene product specific monoclonal antibody, and
   (b) a second container contains a detectably labeled antibody.

16. The kit of claim 15 wherein said antibody is a monclonal antibody with epitopic specificity different from that of the monoclonal antibody of said first container.

17. The kit as in either of claims 12 or 15 wherein said kit also comprises a container containing a disrupting agent.

18. The kit as in either of claims 12 or 15 wherein said kit also comprises a container containing a dissociating agent.

88 12 1893.7
u.Z.: Y 262 EP
Max-Planck-Gesellschaft
zur Förderung der Wissenschaften e.V.

Fig. 1

Fig. 2

A

Fig. 2

B

Fig. 2

C

input
PC #
polyU #

GDG #
GDG top

v.

p66
p55
p51

p31

p24

p17
p15